# EUROPÄISCHE PATENTSCHRIFT

(11) **EP 1 234 588 B1**
(45) Veröffentlichungstag und Bekanntmachung des Hinweises auf die Patenterteilung: **03.11.2004**
(21) Anmeldenummer: 02002534.2
(22) Anmeldetag: 04.02.2002
(51) Int. Cl.: A61L 27/38, B05D 3/06

(54) **Optisches Verfahren zur Verbesserung der Haftung von biologischen Zellen auf einer Polymeroberfläche**
Optical method for improving the adhesion of biological cells on polymer surfaces
Méthode optique d'amélioration de l'adhérence de cellules biologiques sur des surfaces polymériques

(30) Priorität: 06.02.2001 AT 1842001
(43) Veröffentlichungstag der Anmeldung: 28.08.2002
(73) Patentinhaber: Heitz, Johannes, 4040 Linz (AT)
(72) Erfinder: Heitz, Johannes, 4040 Linz (AT); Gumpenberger, Thomas, 4020 Linz (AT); Huber, Norbert, 4040 Linz (AT); Bäuerle, Dieter, 4203 Altenberg (AT); Pühringer, Josef, 4502 St. Marien (AT); Svorcik, Vaclav, 170 00 Prag (CZ); Walachova, Katerina, 701 00 Ostrau (CZ)

(56) Entgegenhaltungen:
- KANG, EN TANG ET AL: "Surface modification of fluoropolymers via molecular design" ADVANCED MATERIALS (WEINHEIM, GERMANY) (2000), 12(20), 1481-1494 , 2000, XP002231524
- HEITZ, JOHANNES ET AL: "Surface modification of fluorocarbon polymers by vacuum-UV excimer-- lamp irradiation in reactive gas atmosphere" JAPANESE JOURNAL OF APPLIED PHYSICS, PART 1: REGULAR PAPERS, SHORT NOTES REVIEW PAPERS (1996), 35(7), 4110-4116 , 1996, XP002231525
- JANSEN J A ET AL: "EFFECT OF SURFACE TREATMENTS ON ATTACHMENT AND GROWTH OF EPITHELIALCELLS" BIOMATERIALS, ELSEVIER SCIENCE PUBLISHERS BV., BARKING, GB, Bd. 10, Nr. 9, 1. November 1989 (1989-11-01), Seiten 604-608, XP000081739 ISSN: 0142-9612
- STEELE J G ET AL: "Mechanism of initial attachment of corneal epithelial cells to polymeric surfaces" BIOMATERIALS, ELSEVIER SCIENCE PUBLISHERS BV., BARKING, GB, Bd. 18, Nr. 23, 1. Dezember 1997 (1997-12-01), Seiten 1541-1551, XP004098576 ISSN: 0142-9612
- SVORCIK V ET AL: "MUSCLE CELL ADHESION ON POLYTETRAFLUORETHYLENE MODIFIED BY UV IRRADIATION" JOURNAL OF MATERIALS SCIENCE LETTERS, CHAPMAN AND HALL LTD. LONDON, GB, Bd. 20, Nr. 21, 1. November 2001 (2001-11-01), Seiten 1941-1942, XP001086005 ISSN: 0261-8028

## Beschreibung

Die Erfindung betrifft ein optisches Verfahren zur Verbesserung der Haftung von biologischen Zellen, insbesondere von Endothel- oder Epithelzellen von Säugetieren oder Menschen, auf einer speziell präparierten Polymeroberfläche sowie die Verwendung dieser Oberflächen mit einer darauf haftenden Zellschicht in der Medizin.

Über viele Jahre wurden intensive Forschungsanstrengungen unternommen, um Materialien zu finden, die biologisch und chemisch stabil gegen körperflüssigkeiten sind. Dieses Gebiet ist von immer noch zunehmender Bedeutung für die Entwicklung verschiedener medizinischer Gegenstände, die in direktem Kontakt mit Körperflüssigkeiten oder Körpergewebe kommen wie Kontaktlinsen, intraokulare Linsen, Prothesen, künstliche Blutgefäße und Körpergewebe, Stents, Herzklappen, Brustimplantate, Katheder, Operationsbesteck und so weiter. Es hat sich als vorteilhaft herausgestellt, viele dieser Artikel aus Kunststoffen herzustellen oder sie mit Kunststoffen zu beschichten.

Besonders die Verwendung von fluorhaltigen Polymeren als dünne abweisende und chemisch inerte Beschichtungen auf Implantaten, Prothesen oder medizinischen Instrumenten ist bekannt. So beschreibt die Anmeldung WO 9810806 A1 ein künstliches Blutgefäß. Hier wurde eine überstreckte poröse Membran aus Polytetrafluorethylen (PTFE) benutzt. Die Patentanmeldung WO 9210532 beschreibt ein Verfahren zur Herstellung von künstlichen Blutgefäßen, die innen mit Fluorpolymeren beschichtet sind. Fluorpolymer-Beschichtungen oder Membranen werden auch bei anderen Implantaten in der Herzchirurgie wie Stents eingesetzt, die ebenfalls ständig dem Blutstrom ausgesetzt sind. Die Patentschrift WO 9317077 A1 beschreibt eine mit einer Fluorpolymerschicht versehene Operationsnadel für die Chirurgie. Durch die Beschichtung wird eine verminderte Beschädigung der Zellen im benachbarten Gewebe durch das Implantat erreicht. Neben Fluorpolymeren werden auch oft Silikone verwendet.

Jedoch führt der Einsatz praktisch aller Kunststoffe zu Abwehrreaktionen des Körpers und bei direktem Kontakt zu Blut besteht selbst bei Fluorpolymeren und Silikonen, die blutverträglicher sind als die meisten anderen Kunststoffe, die Gefahr von Ablagerungen. Beide Effekte werden entweder medikamentös unterdrückt oder es werden in die Polymere direkt direkt pharmakologische Wirkstoffe wie Heparin eingebaut, wie dies zum Beispiel in Anmeldung WO 9810806 A1 beschrieben ist.

Als ideale Oberfläche gegenüber Körperflüssigkeiten und Körpergewebe werden seit langer Zeit natürlich dort vorkommende lebende Epithel- oder Endothelzellen angesehen und ein Großteil der aktuellen Forschung in diesem Gebiet konzentriert sich auf die Entwicklung von Verfahren, um solche Zellen gut haftend auf Kunststoffoberflächen anzusiedeln. Obwohl dazu verschiedene Methoden der Oberflächenmodifikation von Kunststoffen, wie sie allgemein zur Verbesserung der Haftung von Beschichtungen eingesetzt werden, untersucht wurden, ist es bisher noch nicht gelungen, ein Verfahren zu finden, das diese Aufgabenstellung optimal löst.

Eine Methode zur Haftungsverbesserung von Beschichtungen auf Kunststoffoberflächen ist die Aufrauung, die entweder aktiv hergestellt oder bereits durch die richtige Wahl des Materials vorhanden ist. So beschreibt US 5,219,361, dass man die Haftung von lebenden Zellen auf einer Kunststoffoberfläche dadurch erhöhen kann, indem man die Oberfläche mit einer spezifischen Mikrorauigkeit versieht. Die Mikrorauigkeit dient dabei dazu, Verankerungspunkte für die Gewebezellen zur Verfügung zu stellen.

Eine andere Methode Kunststoffoberflächen zu verändern ist, bestimmte Monomere oder Oligomere eines anderen, besser verträglichen oder reaktiven Polymers auf der Oberfläche aufzupfropfen. Dazu werden durch Behandlung der Oberfläche z.B. mit Röntgenstrahlung, UV-Licht, Koronaentladung, Glimmentladung, Elektronen- oder Ionenstrahlen Reaktionsstellen in der Oberfläche erzeugt, an die die gewünschten Gruppen kovalent chemisch gebunden werden. So beschreibt US 5,229,172 das Aufpfropfen von Acrylamid, an dem in einem zweiten Schritt verschiedene biofunktionale Moleküle angebracht werden können, die unter anderem die Adhäsion von Zellen sowohl verbessern als auch vermindern können.

Durch ähnliche Methoden werden auch einfache Aminosäuren oder Sequenzen von Aminosäuren an der Polymeroberfläche kovalent chemisch gebunden. So beschreibt zum Beispiel das Patent US 5,278,063, dass Tierzellen vermehrt auf einer Polymeroberfläche haften, auf der eine bestimmte Sequenz von Aminosäuren eingebaut wurde. Die Wirkungsweise dieser Sequenzen aus Aminosäuren beruht wie die der oben genannten Funktionsmoleküle auf biochemischen Prozessen. Deshalb führen andere Sequenzen von Aminosäuren sehr wohl auch zur deutlichen Verschlechterung der Adhäsion von Zellen auf der Oberfläche, was auch interessante Anwendungen zum Beispiel zur Vermeidung unerwünschter Zellanlagerungen hat.

Man kann die Bioaktivität einer Polymeroberfläche auch durch eine Beschichtung der Oberfläche durch Plasmapolymerisation verändern. So beschreibt das Patent US 5,804,263 ein kombiniertes Plasma- und Röntgen-Polymerisationsverfahren, durch das insbesondere intraokulare Linsen aus Polymethylmethacrylat (PMMA) mit einem hydrophilen Polymer beschichtet werden, um so das Klebenbleiben von Endothelzellen der Augenhornhaut an dieser Oberfläche zu verhindern.

In dem Patent US 4,927,676 wird die Behandlung einer Polymeroberfläche durch den Einsatz einer Plasmaentladung, die unter anderem Stickstoffatome enthält, beschrieben. In einer mit einem Sauerstoff-Ammoniak-Plasma behandelten veränderten Polyuretanoberfläche, konnte durch eine ESCA-Analyse eine Erhöhung des Gehalts an Stickstoff und Sauerstoff gegenüber einer unbehandelten Probe beobachtet werden. Auf vorbehandelten Polyurethanproben (PUR-Proben) sowie auf Proben aus Polyethylenterephthalat (PET) oder Polystyren (PS) konnte eine verbesserte Haftung von Endothelzellen festgestellt werden.

Ein weiteres Verfahren zur Veränderung von Polymeroberflächen, so dass Beschichtungen besser haften und sich die Oberflächen besser verkleben lassen, ist die Bestrahlung mit UV-Licht. So beschreibt das Patent EP 0589351 B1 die Verbesserung der Verklebbarkeit von semikristallinen Polymeren durch die Bestrahlung mit dem Licht eines UV-Excimerlasers oder einer UV-Excimerlampe. Der Hauptvorteil dieser optischen Verfahren gegenüber den beschriebenen Plasmaverfahren, der Bestrahlung mit Teilchen und der mechanischen Bearbeitung ist der, dass diese Bearbeitung berührungslos geschieht, da nur das Licht die Probe erreicht, jedoch kein Kontakt zu Materieteilchen besteht. Die Lichtquelle ist typischerweise durch Fenster von der Probe getrennt. Dadurch wird eine Verunreinigung der Probe vermieden und eine leichte Kontrollierbarkeit des Prozesses erreicht. Auch können Verschmutzungen oder reaktive Partikel von der Probe oder des sie umgebenden Mediums die Lichtquelle selbst nicht beschädigen, wogegen speziell bei der Plasmabehandlung die Reaktion der oft aggressiven Gase im Plasma mit den Elektroden ein Problem darstellt. Darüber hinaus kann man durch die Verwendung monochromatischen Lichts, einer geeigneten Optik und Projektions- oder Kontaktmasken die Veränderung des Polymers auf extrem kleine Bereiche beschränken. So ist es in der Mikroelektronik heute Stand der Technik, durch Belichtung eines Fotolacks Strukturen mit einer Strukturgröße von 180 nm zu erzeugen, wie es zum Beispiel in US 6,146,806 und den darin genannten Zitaten beschrieben ist.

Die Bestrahlung mit UV-Licht kann auch in einer reaktiven Atmosphäre durchgeführt werden. In J. Heitz et al.: *Surface Modification of Fluorocarbon Polymers by Vacuum-UV Excimer Lamp Irradiation in Reactive Gas Atmosphere,* Jpn. J. Appl. Phys. **35,** 4110 (1996) wird die Bestrahlung von Oberflächen aus PTFE oder Fluortetrafluorethylen-Kohexafluorpropylen (FEP) mit einer Excimerlampe in einer Ammoniakatmosphäre beschrieben. Die ESCA-Analyse der bestrahlten Proben ergab auch hier eine Erhöhung des Gehalts an Stickstoff und Sauerstoff in der bestrahlten Oberfläche gegenüber unbestrahlten Proben. Die bestrahlten Proben wiesen auch einen deutlich verringerten Wasserkontaktwinkel auf Die behandelte Oberfläche war weniger rau als die unbehandelte Oberfläche und es konnten auf der behandelten Oberfläche Spuren von Ammoniumfluorid nachgewiesen werden. Sowohl Ammoniak als auch das auf der Oberfläche nachgewiesene Ammoniumfluorid sind giftig (siehe zum Beispiel L. Roth: *Sicherheitsfibel Chemie 4. Auflage,* Ecomed Verlagsgesellschaft, Landsberg/Lech , 1985, Seiten 224-228). Insbesondere ist auch die toxische Wirkung dieser Stoffe auf Zellkulturen bekannt. Es ist deshalb nicht zu erwarten, dass das genannte Verfahren die Haftung von lebenden Zellen fördernd unterstützt, zumal auch die beobachtete Glättung der Oberfläche durch dieses Verfahren die mechanische Verankerung der Zellen erschweren sollte.

Zur Herstellung von Polymeroberflächen auf denen lebende Epithel- oder Endothelzellen dauerhaft angesiedelt sind, wäre es sehr wünschenswert, eine optisches Verfahren zur Behandlung von Polymeroberflächen zur Verfügung zu haben, durch das die Oberfläche im bestrahlten Bereich so verändert wird, dass darauf lebende Epithel- oder Endothelzellen gut haften. Der Einsatz des optische Verfahrens würde insbesondere durch die gute räumliche Auflösung eine exakte Kontrolle des Bearbeitungsprozesses erlauben. Ein solches Verfahren ist jedoch nach dem heutigen Stand der Technik nicht verfügbar, da es insbesondere bisher nicht gelungen ist, nachzuweisen, dass die durch Bestrahlung mit Licht an einer Polymeroberfläche erzeugten Modifikationen zu einer verbesserten Haftung von lebenden Zellen führen.

Aufgabe der vorliegenden Erfindung war es deshalb, ein optisches Verfahren zu entwickeln, das die Polymeroberflächen so verändert, so dass darauf lebende Epithel- oder Endothelzellen von Säugetieren oder Menschen gut haftend und dauerhaft angesiedelt werden können und die so beschichteten Polymeroberflächen für Implantate, Prothesen oder Geräte in der Medizin verwendet werden können.

Es wurde gefunden, dass lebende Epithel- oder Endothelzellen von Säugetieren oder Menschen gut haftend auf Polymeroberflächen aufgebracht werden können, wenn die für die Beschichtung vorgesehene Fläche so in einem stickstoffhaltigen oder sauerstoffhaltigen reaktiven Medium mit UV-Licht im Wellenlängenbereich von 120 bis 400 nm bestrahlt wird, dass dadurch in der Polymeroberfläche zusätzliche Stickstoff- oder sauerstoffhaltige Gruppen eingebaut werden, und die Polymeroberfläche anschließend in heißem Wasser oder heißem Wasserdampf sterilisiert wird. Die so mit Zellen beschichteten Polymeroberflächen sind für Implantate, Prothesen oder Geräte in der Medizin geeignet.

In einer vorteilhaften Ausgestaltungsform handelt es sich bei der Polymeroberfläche um ein Polymer aus der Gruppe der Polyolefine, der Polyester, der Polyurethane, der Polystyrene, der Polyacryle, der ganz oder teilweise fluorierten Polyolefine, der Silikone oder der Siloxane handelt. In einer besonders vorteilhaften Ausgestaltungsform des erfindungsgemäßen Verfahrens handelt es sich bei der Polymeroberfläche um ein Polymer aus der Gruppe der ganz oder teilweise fluorierten Polyolefine.

In einer vorteilhaften Ausgestaltungsform des erfindungsgemäßen Verfahrens wird die UV-Strahlung durch einen UV-Laser als Strahlungsquelle erzeugt, wobei dieser UV-Laser insbesondere ein Excimer-Laser ist, dessen Wellenlänge 157nm (F₂), 193 nm (ArF), 248 nm (KrF) oder 308 nm (XeCl) beträgt. Solche Excimer-Laser sind bekannt. Bei ihrem Einsatz für das erfindungsgemäße Verfahren beträgt die Repetitionsrate zwischen 1 und 10000 Hz, geeignete Pulslängen bewegen sich zwischen 10 fs und 1 ms.

In einer ebenfalls vorteilhaften Ausgestaltungsform des erfindungsgemäßen Verfahrens wird die UV-Strahlung durch einen inkohärenten UV-Excimerstrahler (Excimerlampe) als Strahlungsquelle erzeugt, wobei insbesondere die Wellenlängen 146 nm (Kr₂), 172 nm (Xe₂), 222 nm (KrCl), 308 nm (XeCl) zur Anwendung kommen.

Die UV-Bestrahlung wird in einem reaktiven Medium durchgeführt, das Stickstoff oder Sauerstoff enthält. In einer vorteilhaften Ausgestaltungsform des erfindungsgemäßen Verfahrens wird die Bestrahlung in einer gasförmigen Atmosphäre, die Ammoniak oder Hydrazin enthält, bei einem Druck von 0,01 mbar bis 2000 mbar durchgeführt. In einer weiteren vorteilhaften Ausgestaltungsform des erfindungsgemäßen Verfahrens wird die Bestrahlung in einer sauerstoffhaltigen gasförmigen Atmosphäre bei einem Druck von 0,01 bis 2000 mbar durchgetUhrt. In einer weiteren vorteilhaften Ausgestaltungsform der vorliegenden Erfindung wird die Bestrahlung in einer gasförmigen Atmosphäre, die ein Gemisch aus Sauerstoff und Ammoniak enthält, bei einem Druck von 0,01 bis 2000 mbar durchgeführt, durchgeführt, wobei das Verhältnis von Sauerstoff zu Ammoniak unterhalb von 1 : 10 liegt. In einer ebenfalls vorteilhaften Ausgestaltungsform wird die Bestrahlung in einer flüssigen wäßrigen Lösung durchgeführt, die Moleküle mit Aminogruppen, von Molekülen mit Aminogruppen abgeleitete Salze, Ammoniumionen oder Alkohole enthält.

Die Sterilisation der Polymeroberflächen in heißem Wasserdampf oder heißem Wasser bei einer Temperatur zwischen 50 °C und 150 °C führt zur Abtötung von Keimen, die sich in dem Zeitraum vor der Bestrahlung oder in dem Zeitraum zwischen der Bestrahlung und der Beschichtung mit lebenden Zellen auf dem Polymermaterial angelagert haben. Der Zeitraum zwischen Bestrahlung und Beschichtung kann dabei viele Tage betragen. Es wurde gefunden, dass der Sterilisationsschritt nicht nur zur Abtötung von Keimen führt, sondern auch die Entfernung giftige Substanzen, die sich durch den Bestrahlungsprozess auf der Oberfläche abgelagert haben, bewirkt.

In einer weiteren vorteilhaften Ausgestaltungsform des erfindungsgemäßen Verfahrens werden die bestrahlten Polymeroberflächen noch vor der Sterilisation mit einer Lösung in Kontakt gebracht, die Aminosäuren, Peptide oder biofunktionale Moleküle enthält. Diese Gruppen können an bei der Bestrahlung in der Polymeroberfläche entstandenen Radikalen und Doppelbindungen kovalent verankert werden. Diese biochemisch aktiven Gruppen können die Haftung von lebenden Epithel- oder Endothelzellen weiter fördern oder Polymeroberflächen mit zusätzlichen Biofunktionen versehen.

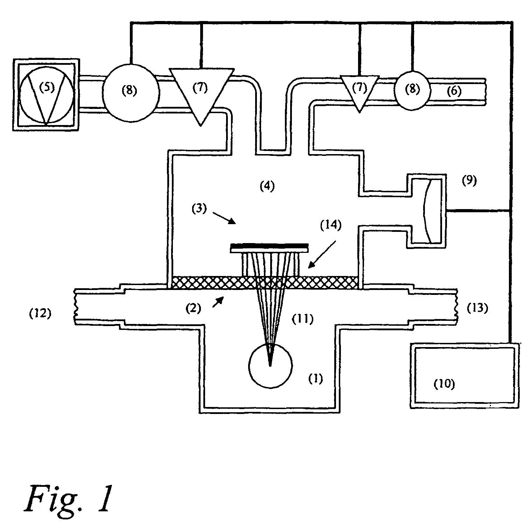

Figur 1 zeigt schematisch die Bestrahlung einer Polymeroberfläche in einer vorteilhaften Ausgestaltungsform des erfindungsgemäßen Verfahrens. Eine UV-Lichtquelle (1), bei der es sich um einen UV-Excimerlaser oder um eine UV-Excimerlampe handelt, strahlt Licht im Wellenlängenbereich von 120 - 400 nm ab. Durch ein Fenster (2), das für das UV-Licht transparent ist wird die Polymerprobe (3), die sich in einer Reaktionskammer (4) befindet, auf der zu beschichtenden Fläche beleuchtet. Die Reaktionskammer ist mit einer Verbindung zu einer Pumpe (5) und Einlässen (6) für das reaktive Medium versehen. Sowohl die Verbindung zur Pumpe als auch die Einlässe sind mit regelbaren Ventilen (7) und Durchflussmessern (8) versehen. Zusätzlich sind an der Kammer ein oder mehrere Sensoren (9) angebracht, mit denen zum Beispiel der Druck in der Reaktionskammer gemessen wird. Die Durchflussmesser, die Sensoren und die Ventile sind mit einer Steuereinheit (10) verbundenen. Der optische Weg von der Lichtquelle zum Fenster (11) ist gasdicht abgeschlossen und mit einem Spülgasanschluss (12) und Spülgasabfluss (13) versehen, so dass bei Verwendung von Licht mit einer Wellenlänge unterhalb von 190 nm der optische Gas (zum Beispiel Stickstoff oder Argon) gespült werden kann oder mit Hilfe einer Vakuumpumpe abgepumpt werden kann. Die Probe ist auf einem Probenhalter (14) befestigt, der durch entsprechende Abdeckungen und Aussparungen gleichzeitig als Kontaktmaske dienen kann. Es versteht sich, dass die Kammer zusätzlich mit Verschiebeeinrichtungen versehen sein kann, so dass eine Probe an mehreren Stellen bestrahlt werden kann, oder ein kontinuierlicher Prozess möglich ist.

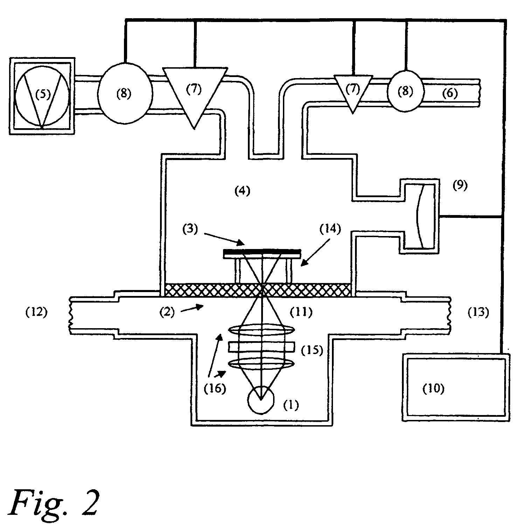

Figur 2 zeigt schematisch die Bestrahlung einer Polymeroberfläche in einer weiteren vorteilhaften Ausgestaltungsform des erfindungsgemäßen Verfahrens. Der Aufbau entspricht ebenso wie die Nummerierung weitgehend der Konstruktion in Figur 1. In den optischen Weg von der Lichtquelle zum Fenster sind zusätzlich eine Projektionsmaske (15) und eine Abbildungsoptik (16) eingebaut, die die Projektionsmaske auf die Probe abbildet.

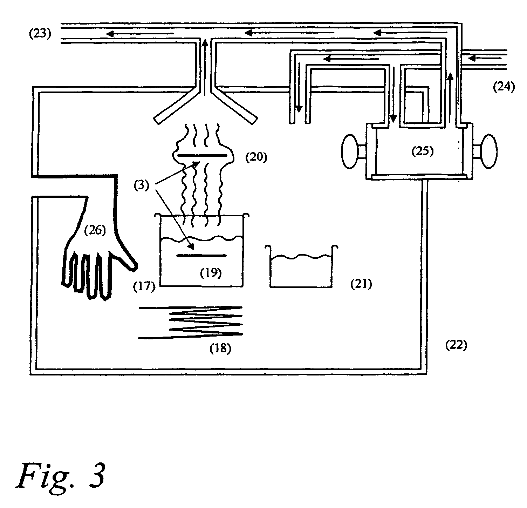

Figur 3 zeigt schematisch eine mögliche Einrichtung zur erfindungsgemäßen Sterilisation und Beschichtung der Polymeroberflächen. Ein gefüllter Wasserbehälter (17) wird mit Hilfe einer Heizvorrichtung (18) erhitzt. Die Probe (3) wird entweder auf einem Probenhalter im Wasserbad (19) oder oberhalb des Wasserbads (20) angebracht und durch das heiße Wasser oder den heißen Wasserdampf sterilisiert. Nach der Sterilisation werden die Polymeroberflächen zur Beschichtung in ein weiteres Behältnis (21) gegeben, das mit einer Lösung gefüllt ist, in der sich die lebenden Zellen in suspendierter Form befinden. Beide Anlagen befinden sich in einer sterilen Glovebox (22), die mit einer Abzugvorrichtung (23), einer sterilen Gasversorgung (24) und Materialschleusen (25) ausgerüstet ist. Zur Handhabung der Proben ist die Glovebox mit Gummihandschuhen (26) versehen. Nach der Beschichtung werden die Polymerproben entweder zusammen mit einem entsprechenden Nährmedium steril verpackt und gelagert oder sofort verwendet.

In dem erfindungsgemäßen Verfahren werden die Polymeroberflächen in einem reaktiven Medium, das Stickstoff oder Sauerstoff enthält, bestrahlt. Dazu werden die Polymerproben auf einem Probenhalter befestigt, der in eine Reaktionskammer eingebaut wird. Die Reaktionskammer wird mit dem Reaktionsmedium, das entweder Stickstoff- oder Sauerstoffverbindungen oder beides enthält, bei den gewünschten Reaktionsbedingungen gefüllt. Mit Hilfe der regelbaren Ventile und der Pumpe wird ein konstanter Durchfluss in der Kammer eingestellt, wobei der Bestrahlungsvorgang auch ohne Durchfluss also mit geschlossenen Ventilen durchgeführt werden kann. Anschließend wird die UV-Lichtquelle eingeschaltet und die Polymeroberfläche für die vorher festgelegte Bestrahlungszeit der UV-Strahlung ausgesetzt. Die UV-Strahlung bewirkt durch Fotodissoziation, dass in dem reaktiven Medium neue Spezies entstehen, die mit der Polymeroberfläche reagieren. Dadurch werden in der Polymeroberfläche neue stickstoffhaltige oder sauerstoffhaltige Gruppen eingebaut. Die Reaktion ist praktisch auf die bestrahlten Bereiche beschränkt, da die entstehenden Spezies sehr reaktiv sind und dann besonders effektiv zur Oberfläche gelangen, wenn sie nahe der Oberfläche erzeugt werden. Durch die Bestrahlung kommt es auch im Polymer selbst zu Fotoreaktionen. Es entstehen zum Beispiel Radikale und neue Doppelbindungen. Diese neuen Gruppen können als Reaktionsplätze für die im reaktiven Medium enthalten Spezies dienen. Normalerweise werden nicht alle diese neuen Reaktionsplätze abgesättigt, so dass es nach Entfernung der Probe aus der Reaktionskammer zur Reaktion mit dem Luftsauerstoff kommen kann und man diese Reaktionsplätze zur kovalenten Verankerung weiterer biofunktionaler Gruppen benutzen kann, indem man die Oberfläche möglichst unmittelbar nach dem Bestrahlungsvorgang mit in Lösung befindlichen Wirkstoffen in Kontakt bringt. Die Oberflächenmodifikation ist zumindest über einen Zeitraum von mehreren Wochen stabil. Bei der Bestrahlung entstehen neben kovalent an der Polymeroberfläche gebundenen Gruppen auch Stoffe, die sich auf der Oberfläche nur ablagern. Diese zum Teil giftigen Stoffe können durch den Sterilisationsprozess in hinreichendem Maße entfernt werden. Zur Sterilisationen werden die Polymeroberflächen eine bestimmte Zeitspanne heißem Wasser oder heißem Wasserdampf ausgesetzt. Durch diesen Prozess werden auch auf der Oberfläche vorhandene Keime abgetötet. Nach dem Sterilisationsprozess werden die Polymeroberflächen mit in Lösung suspendierten lebenden Epithel- oder Endothelzellen in Verbindung gebracht. Diese Zellen werden nach bekannten biochemischen Verfahren aus Gewebeproben hergestellt. Die in Suspension nahezu runden Zellen lagern sich auf der modifizierten Polymeroberfläche an und nehmen eine amöbenhafte flache Form an. Die Polymeroberflächen können, nachdem sich eine genügend große Zahl von Zellen auf der Oberfläche angesiedelt hat, nun der Lösung entnommen werden und direkt zu medizinischen Gegenständen wie Implantaten, Prothesen oder Geräten verarbeitet oder als solche eingesetzt werden. Alternativ dazu können die Polymeroberflächen zusammen mit einem geeigneten Medium gelagert werden, so dass sich an der Polymeroberfläche befindlichen Zellen durch Teilung vermehren. Sowohl der Haftungs- als auch der Teilungsprozess können durch zusätzlich an der Oberfläche angebrachte biofunktionale Wirkstoffe gefördert und gesteuert werden. Solche Wirkstoffe können auch dazu dienen, den mit Zellen besiedelten Oberflächen zusätzliche gewünschte biofunktionale Funktionen zu verleihen. Nach dem Sterilisationsschritt muß auf eine sterile Arbeitsumgebung werden. Zweckmäßigerweise wird man deshalb alle weiteren Schritte auf kleinem Raum zum Beispiel in einer Glovebox durchführen.

Die Erfindung sei anhand folgender Beispiele näher erläutert.

### Beispiel 1

Zwei PTFE-Proben werden nach dem erfindungsgemäßen Verfahren mit einer Xe₂-Excimerlampe bei einer Wellenlänge von 172 +/- 5 nm und einer Leistungsdichte in der Größenordnung von 100 mW/cm² in einer gasförmigen Ammoniakatmosphäre bei einem Druck von 5 mbar beidseitig für je 10 und 20 Minuten bestrahlt. Mehrere Wochen nach der Bestrahlung wurde der Wasserkontaktwinkel gemessen und mit einer unbestrahlten PTFE-Probe verglichen. Die unbestrahlte Probe hatte einen Wasserkontaktwinkel gegenüber deionisiertem Wasser von 116°, die 10 Minuten lang bestrahlte Probe hatte dagegen einen Kontaktwinkel von 72° und die für 20 Minuten lang bestrahlte Probe einen Kontaktwinkel von 67°. Aus dem Rückgang des Kontaktwinkel kann auf den Einbau neuer Gruppen an die Polymeroberfläche geschlossen werden.

### Beispiel 2

Zwei PTFE-Proben werden nach dem erfindungsgemäßen Verfahren mit einer Xe₂-Excimerlampe bei einer Wellenlänge von 172 +/- 5 nm und einer Leistungsdichte in der Größenordnung von 100 mW/cm² in einer gasförmigen Sauerstoffatmosphäre bei einem Druck von 5 mbar beidseitig für je 10 Minuten bestrahlt. Mehrere Wochen nach der Bestrahlung wurde der Wasserkontaktwinkel gemessen und mit einer unbestrahlten PTFE-Probe verglichen. Die unbestrahlte Probe hatte einen Wasserkontaktwinkel gegenüber deionisiertem Wasser von 116°, die 10 Minuten lang bestrahlte Probe hatte dagegen einen Kontaktwinkel von 94°. Aus dem Rückgang des Kontaktwinkel kann auf den Einbau neuer Gruppen an der Polymeroberfläche geschlossen werden.

### Beispiel 3

Vier PTFE-Proben werden nach dem erfindungsgemäßen Verfahren mit einer Xe₂-Excimerlampe bei einer Wellenlänge von 172 +/- 5 nm und einer Leistungsdichte in der Größenordnung von 100 mW/cm² in einer gasförmigen Ammoniakatmosphäre bei einem Druck von 5 mbar beidseitig für je 5 und 10 Minuten bestrahlt. Die Hälfte der Proben wird gleich nach der Bestrahlung für etwa 12 Stunden einer wäßrigen Lösung der Aminosäure Alanin ausgesetzt. Mehrere Wochen nach der Bestrahlung wurde mit einer anderen Apparatur als in Beispiel 1 der Wasserkontaktwinkel gemessen (weshalb die Meßergebnisse etwas variieren) und mit einer unbestrahlten PTFE-Probe verglichen. Die unbestrahlte Probe hatte einen "advancing" Wasserkontaktwinkel gegenüber deionisiertem Wasser von 108°. Von den beiden Proben, die nicht mit der gelösten Aminosäure in Kontakt kamen, hatte die 5 Minuten lang bestrahlte Probe einen Kontaktwinkel von 71° und die für 10 Minuten lang bestrahlte Probe einen Kontaktwinkel von 61°. Dagegen hatte von den beiden Proben, die mit der gelösten Aminosäure in Kontakt kamen, die 5 Minuten lang bestrahlte Probe einen Kontaktwinkel von 60° und die 10 Minuten bestrahlte Probe einen Kontaktwinkel von 50°. Aus dem zusätzliche Rückgang des Kontaktwinkels kann geschlossen werden, das an freien Reaktionsplätzen in der Polymeroberfläche bei dem nach erfindungsgemäßen Verfahren modifizierten Polymeroberfläche zusätzlich Aminosäuren kovalent gebunden werden.

### Beispiel 4

Drei weitere PTFE-Proben werden nach dem erfindungsgemäßen Verfahren mit einem ArF-Excimerlaser bei einer Wellenlänge von 193 +/- 1 nm und einer Leistungsdichte von 105 mW/cm² in einer wäßrigen 0,25%-Glutaminsäurelösung bei einem PH-Wert zwischen 8 und 9,5 für je 20 Sekunden, 60 Sekunden und 200 Sekunden bestrahlt. Nach der Bestrahlung wurde der Wasserkontaktwinkel mit der gleichen Einrichtung wie in Beispiel 3 gemessen und mit einer unbestrahlten PTFE-Probe verglichen. Die unbestrahlte Probe hatte einen "advancing" Wasserkontaktwinkel gegenüber deionisiertem Wasser von 112°, die 20 Sekunden lang bestrahlte Probe hatte dagegen einen Kontaktwinkel von 77°, die für 60 Sekunden lang bestrahlte Probe einen Kontaktwinkel von 65° und die für 200 Sekunden bestrahlte Probe einen Kontaktwinkel von 53°. Aus dem Rückgang des Kontaktwinkel kann auch hier auf den Einbau neuer Gruppen an die Polymeroberfläche geschlossen werden.

### Beispiel 5

Vier weitere PTFE-Proben wurden unter den gleichen Bedingungen wie in Beispiel 1 nach dem erfindungsgemäßen Verfahren für 5, 10, 15 und 20 Minuten bestrahlt. Mehrere Wochen nach der Bestrahlung wurden sie zusammen mit einer unbestrahlten Probe nach dem erfindungsgemäßen Verfahren in heißem Wasserdampf sterilisiert. Direkt anschließend wurden die Polymeroberflächen mit einer geeigneten Nährlösung bedeckt, in der menschliche Hautzellen (Keratinozyten) suspendiert waren. Die Proben verblieben für 24 Stunden in der Nährlösung. Anschließend wurde die überstehende Nährlösung abgeschüttet und durch neue Nährlösung ersetzt. Dann wurden die Zellen von der Oberfläche mit dem Enzym Trypsin abgelöst, mit Farbstoff eingefärbt und unter dem Mikroskop in einer Burker-Kammer ausgezählt. Der Bedeckungsgrad der unbestrahlten PTFE-Probe betrug 3.000 Zellen pro cm², die Probe mit einer Bestrahlungszeit von 5 Minuten war mit 9.000 Zellen pro cm² bedeckt, die Probe mit 10 Minuten mit 12.000 Zellen pro cm², die Probe mit 15 Minuten mit 13.500 Zellen pro cm² und die Probe mit 20 Minuten mit 22.500 Zellen pro cm². Man sieht, dass diese Epithelzellen auf der nach dem erfindungsgemäßen Verfahren hergestellten Oberfläche sehr gut haften.

### Beispiel 6

Vier weitere PTFE-Proben wurden unter den gleichen Bedingungen wie in Beispiel 1 nach dem erfindungsgemäßen Verfahren für 5, 10, 15 und 20 Minuten bestrahlt. Direkt nach der Bestrahlung wurden sie für 12 Stunden in eine wässrige Lösung der Aminosäure Alanin eingelegt. Dann wurden sie in gleicher Weise wie in Beispiel 5 mit Keratinozyten besiedelt und die Zellhaftung mit der gleichen Methode wie zuvor ausgewertet. Die Probe mit einer Bestrahlungszeit von 5 Minuten war mit 10.500 Zellen pro cm² bedeckt, die Probe mit 10 Minuten mit 15.000 Zellen pro cm², die Probe mit 15 Minuten mit 28.500 Zellen pro cm² und die Probe mit 20 Minuten mit 37.500 Zellen pro cm². Man sieht, dass sich die Haftung der Zellen auf der nach dem erfindungsgemäßen Verfahren hergestellten Oberfläche durch eine zusätzliche Behandlung in einer Alaninlösung deutlich verbessern lässt.

### Beispiel 7

Zwei weitere PTFE-Proben wurden unter den gleichen Bedingungen wie in Beispiel 1 nach dem erfindungsgemäßen Verfahren für 20 Minuten bestrahlt. Direkt nach der Bestrahlung wurde eine der beiden Proben für 12 Stunden in eine wässrige Lösung der Aminosäure Alanin eingelegt. Mehrere Wochen nach der Bestrahlung wurden sie zusammen mit einer unbestrahlten Probe nach dem erfindungsgemäßen Verfahren in heißem Wasserdampf sterilisiert. Direkt anschließend wurden die Polymeroberflächen mit einer geeigneten Nährlösung bedeckt, in der Bindegewebszellen von Mäusen (3T3 Fibroblasten) suspendiert waren. Die Proben verblieben für 24 Stunden in der Nährlösung. Anschließend wurden die Zellen auf der Oberfläche fixiert, mit Toluidin-Blau eingefärbt und unter dem Polarisationsmikroskop bei einer hundertfachen Vergrößerung ausgezählt. Der Bedeckungsgrad der unbestrahlten PTFE-Probe betrug 11.550 Zellen pro cm², die Probe mit einer Bestrahlungszeit von 20 Minuten war mit 46.500 Zellen pro cm² bedeckt und die Probe mit 20 Minuten Bestrahlungszeit und anschließender Alaninbehandlung war mit 79.600 Zellen pro cm² bedeckt. Man sieht, dass sich auch die Haftung der Fibroblasten auf PTFE-Oberflächen durch das erfindungsgemäßen Verfahren signifikant verbessern lässt, insbesondere wenn die Probe direkt nach der Bestrahlung mit einer Aminosäurelösung behandelt wird.

## Patentansprüche

1. Verfahren zur Bearbeitung von Polymeroberflächen, **dadurch gekennzeichnet, dass** die Polymeroberfläche so in einem Stickstoff- oder sauerstoffhaltigen reaktiven Medium mit UV-Licht im Wellenlängenbereich von 120 bis 400 nm bestrahlt wird, so dass dadurch in der Polymeroberfläche zusätzliche Stickstoff- oder sauerstoffhaltige Gruppen eingebaut werden, die Polymeroberfläche dann in heißem Wasser oder in heißern Wasserdampf bei einer Temperatur zwischen 50 °C und 150 °C sterilisiert wird und anschließend lebende Epithel- und Endothelzellen direkt auf die Oberfläche aufgebracht werden.

2. Verfahren gemäß Anspruch 1 **dadurch gekennzeichnet, dass** es sich bei dem Polymer der Polymeroberfläche um ein Mitglied der Gruppe der Polyolefine, der Polyester, der Polyurethane, der Polystyrene, der Polyacryle, der ganz oder teilweise fluorierten Polyolefine, der Silikone oder der Siloxane handelt.

3. Verfahren gemäß Anspruch 1 **dadurch gekennzeichnet, dass** die UV-Strahlung durch einen UV-Excimerlaser mit einer Repetitionsrate zwischen 1 und 10000 Hz und einer Pulslänge von 10 fs und 1 ms erzeugt wird.

4. Verfahren gemäß Anspruch 1 **dadurch gekennzeichnet, dass** die UV-Strahlung durch einen UV-Excimerstrahler erzeugt wird.

5. Verfahren gemäß Anspruch 1 **dadurch gekennzeichnet, dass** die UV-Bestrahlung in einer gasförmigen Atmosphäre, die Ammoniak oder Hydrazin enthält, bei einem Druck von 0,01 bis 2000 mbar durchgeführt wird.

6. Verfahren gemäß Anspruch 1 **dadurch gekennzeichnet, dass** die UV-Bestrahlung in einer gasförmigen sauerstofihaltigen Atmosphäre bei einem Druck von 0,01 bis 2000 mbar durchgeführt wird.

7. Verfahren gemäß Anspruch 1 **dadurch gekennzeichnet, dass** die UV-Bestrahlung in einer gasförmigen Atmosphäre, die ein Gemisch aus Sauerstoff und Ammoniak enthält, bei einem Druck von 0,01 bis 2000 mbar durchgeführt, wobei das Verhältnis von Sauerstoff zu Ammoniak unterhalb von 1 : 10 liegt.

8. Verfahren gemäß Anspruch 1 **dadurch gekennzeichnet, dass** die UV-Bestrahlung in einer wäßrigen Lösung durchgeführt wird, die Moleküle mit Aminogruppen, von Molekülen mit Aminogruppen abgeleitete Salze, Ammoniumionen oder Alkohole enthält.

9. Verfahren gemäß Anspruch 1 **dadurch gekennzeichnet, dass** die bestrahlten Polymeroberflächen noch vor der Sterilisation mit einer Lösung in Kontakt gebracht wird, die Aminosäuren, Peptide, oder biofunktionale Moleküle enthält, und diese Gruppen an der Polymeroberfläche kovalent verankert werden.

10. Implantate, Prothesen, oder Geräte für die Medizin, zu deren Herstellung Polymere benutzt werden, **dadurch gekennzeichnet, dass** die Oberflächen der Polymere durch eines der in den Ansprüchen 1 bis 9 beschriebenen Verfahren behandelt wurden.

## Claims

1. Method for processing polymer surfaces, **characterized in that** the polymer surface is irradiated in a nitrogen- or oxygen-containing reactive medium with UV light in the wavelength range from 120 to 400 nm in such a way that additional nitrogen- or oxygen-containing groups are incorporated into the polymer surface, the polymer surface is then sterilized in hot water or in superheated steam at a temperature between 50°C and 150°C, and subsequently living epithelial and endothelial cells are directly applied to the surface.

2. Method according to Claim 1, **characterized in that** the polymer of the polymer surface is a member of the group of polyolefins, of polyesters, of polyurethanes, of polystyrenes, of polyacrylics, of wholly or partly fluorinated polyolefins, of silicones or of siloxanes.

3. Method according to Claim 1, **characterized in that** the UV radiation is generated by a UV excimer laser with a repetition rate of between 1 and 10 000 Hz and a pulse length of 10 fs and 1 ms.

4. Method according to Claim 1, **characterized in that** the UV radiation is generated by a UV excimer lamp.

5. Method according to Claim 1, **characterized in that** the UV irradiation is carried out in a gaseous atmosphere comprising ammonia or hydrazine under a pressure of from 0.01 to 2000 mbar.

6. Method according to Claim 1, **characterized in that** the UV irradiation is carried out in a gaseous oxygen-containing atmosphere under a pressure of from 0.01 to 2000 mbar.

7. Method according to Claim 1, **characterized in that** the UV irradiation is carried out in a gaseous atmosphere comprising a mixture of oxygen and ammonia under a pressure of from 0.01 to 2000 mbar, where the ratio of oxygen to ammonia is below 1:10.

8. Method according to Claim 1, **characterized in that** the UV irradiation is carried out in an aqueous solution comprising molecules having amino acids, salts derived from molecules having amino groups, or ammonium ions or alcohols.

9. Method according to Claim 1, **characterized in that** the irradiated polymer surfaces are, before the sterilization, brought into contact with a solution comprising amino acids, peptides or bifunctional molecules, and these groups are covalently tethered to the polymer surface.

10. Implants, prostheses, or instruments for medicine, for the production of which polymers are used, **characterized in that** the surfaces of the polymers have been treated by one of the methods described in Claims 1 to 9.

## Revendications

1. Procédé de traitement de surfaces polymères, **caractérisé en ce que** la surface polymère est irradiée dans un milieu réactif, contenant de l'azote ou de l'oxygène, à l'aide de lumière UV dans le domaine de longueurs d'onde de 120 à 400 nm, de manière à ce qu'il y ait ainsi incorporation de groupements supplémentaires, contenant de l'azote ou de l'oxygène, dans la surface polymère, **en ce que** la surface polymère est alors stérilisée dans de l'eau chaude ou dans de la vapeur d'eau chaude, à une température comprise entre 50°C et 150°C, et **en ce que** des cellules épithéliales et des cellules endothéliales vivantes sont ensuite appliquées directement sur la surface.

2. Procédé selon la revendication 1, **caractérisé en ce qu'**il s'agit, dans le cas du polymère de la surface polymère, d'un membre du groupe des polyoléfines, des polyesters, des polyuréthannes, des polystyrènes, des résines polyacryliques, des polyoléfines complètement ou partiellement fluorées, des silicones ou des siloxanes.

3. Procédé selon la revendication 1, **caractérisé en ce que** le rayonnement UV est produit par un laser excimère UV ayant une vitesse de répétition comprise ente 1 et 10000 Hz et une durée de pulsion comprise entre 10 fs et 1 ms.

4. Procédé selon la revendication 1, **caractérisé en ce que** le rayonnement UV est produit par un dispositif de rayonnement à laser excimère UV.

5. Procédé selon la revendication 1, **caractérisé en ce que** le rayonnement UV est effectué dans une atmosphère gazeuse, qui contient de l'ammoniac ou de l'hydrazine, à une pression de 0,01 à 2000 mbars.

6. Procédé selon la revendication 1, **caractérisé en ce que** le rayonnement UV est effectué dans une atmosphère gazeuse, contenant de l'oxygène, à une pression de 0,01 à 2000 mbars.

7. Procédé selon la revendication 1, **caractérisé en ce que** le rayonnement UV est effectué dans une atmosphère gazeuse, qui contient un mélange d'oxygène et d'ammoniac, à une pression de 0,01 à 2000 mbars, le rapport de l'oxygène à l'ammoniac étant inférieur à 1:10.

8. Procédé selon la revendication 1, **caractérisé en ce que** le rayonnement UV est effectué dans une solution aqueuse qui contient des molécules ayant des groupements amino, des sels dérivés de molécules ayant des groupements amino, des ions ammonium ou des alcools.

9. Procédé selon la revendication 1, **caractérisé en ce que** les surfaces polymères irradiées sont mises en contact, encore avant la stérilisation, avec une solution qui contient des acides aminés, des peptides ou des molécules bio-fonctionnelles et **en ce que** ces groupements sont ancrés d'une manière covalente à la surface polymère.

10. Implants, prothèses ou appareils pour la médecine, en vue de la fabrication desquels on utilise des polymères, **caractérisés en ce que** les surfaces des polymères ont été traitées par l'un des procédés décrits dans les revendications 1 à 9.
